Europäisches Patentamt

European Patent Office

Office européen des brevets

(19)

(11) Publication number: **0 245 994**

**A2**

(12) **EUROPEAN PATENT APPLICATION**

(21) Application number: **87303764.2**

(22) Date of filing: **28.04.87**

(51) Int. Cl.4: **B01L 3/14 , G01N 33/53**

(30) Priority: **28.04.86 AU 5660/86**

(43) Date of publication of application:
**19.11.87 Bulletin 87/47**

(84) Designated Contracting States:
**DE FR GB IT**

(71) Applicant: **ICI AUSTRALIA LIMITED**
**1, Nicholson Street**
**Melbourne Victoria 3000(AU)**

(72) Inventor: **McCaskill, Alistair**
**17 Clifton street**
**North Balwyn, Victoria(AU)**
Inventor: **Lichti, Gottfried**
**49 Cooper street**
**Essendon, Victoria(AU)**

(74) Representative: **Jennings, Nigel Robin et al**
**KILBURN & STRODE 30 John Street**
**London WC1N 2DD(GB)**

(54) **Method and apparatus for reaction.**

(57) A reaction vessel comprises a first body (1) defining an open cavity adapted to receive a portion of a second body (21). The surface of the cavity comprises a first portion (2) adjacent the exterior of the cavity which conically tapers inwards with an included angle of about 6° and a second portion (3) which conically tapers inwards with an included angle of about 90°. The external surface of the second body (21) includes a first portion (22) which conically tapers with an included angle of about 6° and a second portion (23) with an included angle of about 90°. The surface (22) of the second body (21) carries a plurality of short projections (26). The shape of the second body substantially matches that of the cavity in the first body whereby when the one is inserted into the other a capillary space having a thickness of about 0.05mm is defined between them. The reaction vessel is prepared for use by immobilizing one or more reactants on one or more of the surfaces (2) and (22).

FIG. 3.

# METHOD AND APPARATUS FOR REACTION

This invention relates to method and apparatus for reaction.

In particular aspects it includes reaction vessels and kits including a solid phase for chemical and biological reactions, assemblages of such kits and methods for the use of such kits.

The invention has particular application in chemical and biological analysis both qualitative and quantitative.

Solid phase reactions are well known and for example have been used widely in immunoassay. Conventional solid phase reaction techniques use microtitre plate wells as the solid phase support to which a reactant has been immobilized by adsorption or covalent bonding. A co-reactant which may be a liquid, or material dissolved or suspended in a liquid is then placed in the wells. One of the major limitations to the use of such techniques is the long reaction times required. A factor in the long reaction times may be that the co-reactants must diffuse a considerable distance in the well to react with the reactant immobilized on the walls of the well.

In an attempt to overcome the problem of long reaction times it has been proposed to use capillary tubes in which a reactant is immobilized on the inner wall. The small diameters of capillary tubes ensures that diffusion of co-reactants is only required to take place over relatively small distances. There are, however, disadvantages with capillaries, in particular the difficulty on a commercial scale of carrying out the requisite reactions to immobilize reactant on the inner wall surface. This is particularly the case with biologically reactive reactants.

Other difficulties are found in using capillary tubes including the difficulty of reproducibly forming a capillary of dimensions less than 1 mm, the difficulty of joining a capillary tube to other apparatus, the difficulty that a capillary tube has a small surface area and small volume and the difficulty of producing homogenous coatings within a capillary tube.

Accordingly, the present invention seeks to provide a reaction vessel which can be assembled to form a capillary space.

The present invention provides a reaction vessel comprising:
a first body having a cavity therein extending to the exterior thereof and
a second body having a portion adapted to be received in said cavity,
wherein said cavity conically taperingly narrows towards the inner end of the cavity with an included conic angle of less than 140°,
wherein said portion conically taperingly narrows towards an end of said second body with an included conic angle of less than 140°,
wherein the cavity and said portion are so similarly shaped as to define,
and wherein one of the cavity and said portion has projections thereon adapted to space apart said first body and said second body when said second body is received within said cavity so as to define,
when said portion is in a juxtaposition within said cavity,
a capillary space between said first body and said second body having a thickness of not greater than 1.0 mm.

Preferably, said cavity conically taperingly narrows towards said inner end and said portion conically taperingly narrows towards said end of said second body with an included conic angle of less than 90°, more preferably less than 60°, more preferably less than 30°, more preferably less than 15°, still more preferably less than 10° and most preferably less than 7½°.

Said capillary space preferably has a thickness not greater than 0.6mm, more preferably 0.2 mm and most preferably not greater than 0.05mm.

Preferably said capillary space has a volume, when in use, of not more than 500, more preferably 200 and most preferably 50 microlitres.

In a preferred instance the present invention provides a reaction vessel comprising a first body having a cavity therein extending to the exterior thereof and a second body having a portion adapted to be received in said cavity,
wherein the cavity has a first part adjacent the exterior of the first body which conically taperingly narrows towards the inner end of the cavity with an included conic angle of less than 140°,
and a second part extending to the inner end of the cavity which conically taperingly narrows towards the inner end of the cavity with an included conic angle of from 30° to 175°,
wherein said portion has a first part remote from an end of said portion which conically taperingly narrows towards said end of said portion with an included conic angle of less than 140°,
and a second part extending to said end of said portion which conically taperingly narrows towards said end of said portion with an included conic angle of from 30° to 175°,
wherein the first part of said cavity and said first part of said portion are so similarly shaped such as to define,
and wherein one of said first part of said cavity and said first part of said portion has projections thereon adapted to space apart said first part of said cavity and said first part of said portion when said

2

portion is received within said cavity so as to define,

when said portion is in a juxtaposition within said cavity,

a capillary space between said first part of the cavity and said first part of said portion having a thickness of not greater than 1.0 mm.

Preferably said first part of said portion and said first part of said cavity each have a surface area of from 1 to 20cm$^2$ more preferably 1 to 8cm$^2$.

Preferably said first part of said cavity conically taperingly narrows towards the inner end of said cavity and said first part of said portion conically taperingly narrows towards said end of said portion with an included conic angle of less than 90°, more preferably less than 60, more preferably less than 30°, more preferably less than 15°, and still more preferably less than 10° and most preferably less than 7½°.

Preferably, said second part of said cavity conically taperingly narrows towards the inner end of said cavity and said second part of said portion conically taperingly narrows towards said end of said portion with an included angle of from 60° to 140°, more preferably 70° to 120°, more preferably from 80° to 100° and most preferably about 90°.

The conic angle of said first part of said cavity and said first part of said portion is preferably less than the conic angle of said second part of said cavity and said second part of said portion.

Preferably the inner end of said cavity and said end of said portion are generally frusto-conic or rounded and thereat have a diameter of from 0.5 to 2.5 mm.

Said projections are preferably upstanding by an amount of not greater than 1.0 mm, more preferably 0.6mm, more preferably 0.2mm and most preferably 0.05mm.

We have found it most convenient if said projections are located on said first part of said portion.

Said vessel is preferably made by injection moulding of synthetic plastics material.

Said vessel or at least said body is preferably made of a transparent material. However, it should be noted that transparency and in particular transparency with respect to visible light may not always be required. In some instance, for particular reactions, said vessel is preferably made of a material which is transparent to one of UV or IR light.

A particularly suitable material for the vessel is polystyrene but this is not to be construed as limiting.

Preferably the first body or the second body is dimensioned such as to be receivable in standard sized test.tube holders or sample vessel holders as used in the chemical or pathological testing industries.

Concerning said conic angle of from 30° to 175°, we have found that if an angle of less than 60° is used in respect of said cavity there is a tendency for an air bubble to be trapped at the inner end of said cavity when liquid is introduced into the first part and if an angle of greater than 140° is used in respect of said second part of said portion there is a tendency for an air bubble to be trapped at said end of said portion when said portion is introduced into liquid in the first part. The alternative use of a single conic part while possible might require an excessively large cross-sectional area for some applications.

We have found that the use of said projections as a means of defining said capillary space and its thickness is superior to relying on said end of said portion abutting against said inner end of the cavity particularly because of tolerance drift in injection moulding, mould sprues and because of dimensional differences with respect to individual cavities as compared to other cavities in multicavity injection moulding dies.

Preferably an overflow space is provided in the first body to receive liquid displaced from said cavity when said second body is introduced into said cavity.

Preferably said second body has a portion adapted to be held by a user to introduce said second body into said cavity.

The present invention also provides a method of carrying out chemical or biological reactions utilizing a reaction vessel in accordance with this invention which comprises locating said second body in said cavity in said juxtaposition to define said capillary space wherein a reactant has been immobilized on one of the surface of said cavity and said second body defining said capillary space prior to them being so juxtaposed and interposing into said capillary space a co-reactant.

In one form the invention provides a kit for chemical or biological reactions, the kit comprising a reaction vessel in accordance with this invention and wherein a reactant is immobilized on one of the surfaces of said cavity and said second body which in use define said capillary space.

In particular aspect the present invention provides an assemblage formed from a kit in accordance with this invention and formed by bringing said surfaces into such juxtaposition to define said capillary space.

Said reactant is preferably immobilized on a solid phase or gel or in any other convenient manner.

A co-reactant may be interposed into said capillary space after formation thereof.

In an alternative, the co-reactant is applied to one of said surface prior to said juxtaposition whereby such interposition occurs in the formation of said capillary space.

More than one said reactant may be immobilized on one of said surfaces. In this last respect, it is probably most convenient if said first part of said portion carries different reactants along its length although the use of different reactants along the length of the cavity is not impossible.

The types of reactions which may be carried out using the method, assemblage or kit of this invention include chemical covalent bonding reactions, inorganic ion reactions including complex formation, elution reactions and biological reactions. Of particular interest are those biological reactions used in immunoassay analysis.

The term capillary space is one which is well understood. For the present invention best results are achieved when the capillary space is such that a substantial number of points in the space are no more than 0.5 mm, more preferably no more than 0.3 mm still more preferably 0.1 mm and most preferably no more than 0.025 mm from a surface.

Techniques for immobilizing reactants are well established and include adsorption and covalent bonding.

An advantage of the present invention arises from the fact that it is relatively easy to immobilize a reactant on a surface prior to assembly, and that reactions occur relatively quickly by the use of a capillary space.

A further advantage of the present invention is that surfaces of the capillary space may have different reactants immobilized on separate surfaces in one capillary space.

To illustrate the present invention a reaction vessel in accordance therewith will now be described with the aid of the accompanying drawings in which:-

Fig. 1 is an elevational view of a first body and a second body of the vessel.

Fig. 2 is a perspective view of the first body and the second body of the vessel, and

Fig. 3 is a cross-sectional view of the vessel.

Fig. 4 is another cross-sectional view of the vessel.

Fig. 5 is a graph representing reactions.

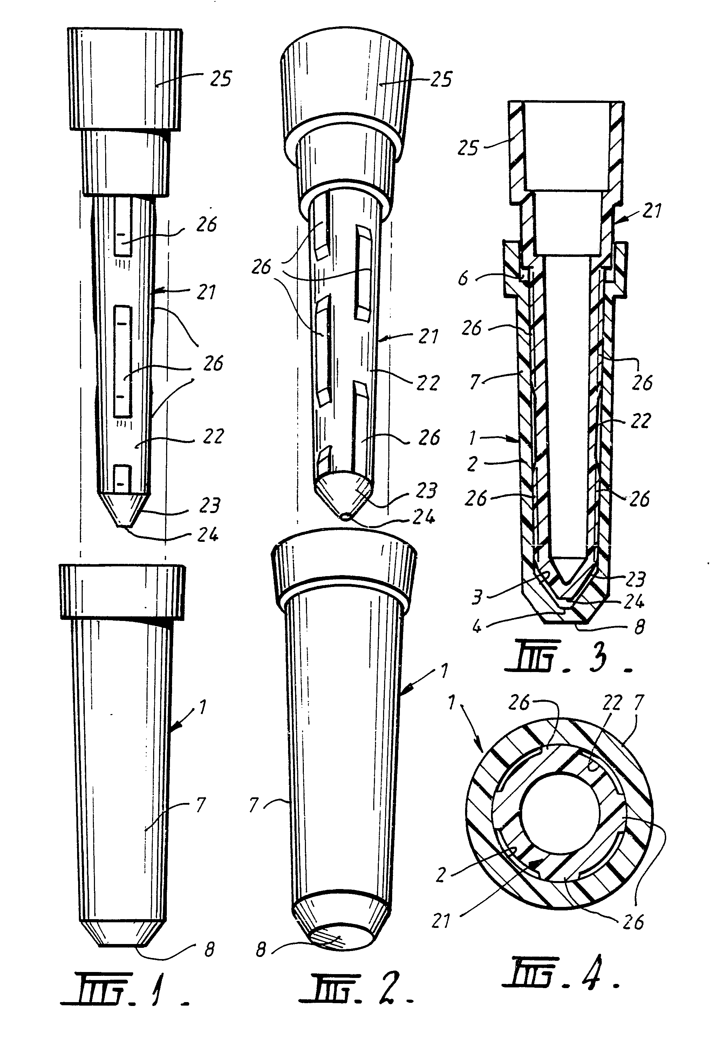

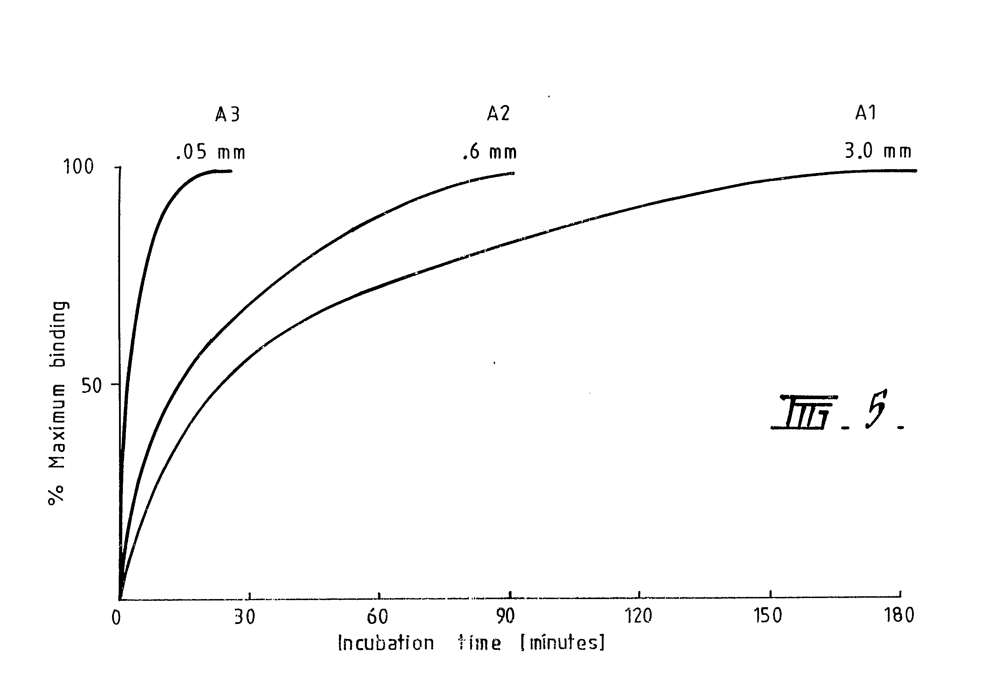

The first body 1 of the reaction vessel shown in Fig 1 and 2 can conveniently be called a bottle and comprises a long conically tapering inner surface 2 of included conic angle of about 6°, a short conically tapering surface 3 of included conic angle of about 90°, a flat bottom 4 having a diameter of about 2mm, an overflow well 6, an outer surface 7 and a base on which the bottle can stand.

The bottle is made of polystyrene.

The second body 21 of the reaction vessel can conveniently be called a plunger and comprises a long conically tapering outer surface 22 of included angle of about 6°, a short conically tapering outer surface 23 of included conic angle of about 90°, a flat end 24 of diameter of about 2mm and an end 25 adapted to be grasped by the fingers of a user or an automatic testing apparatus for the purpose of inserting the plunger into the bottle.

The plunger is also made of polystyrene and is internally hollow.

On the surface 22 of the plunger is a plurality of narrow longitudinally extending projections 26 which extend from the surface 22 a distance of about 0.05mm. In consequence of the projections 26, the surface 22 will locate about 0.05mm away from the surface 2 when the plunger is inserted fully into the bottle. Such location results in a capillary space being formed between the surfaces 2 and 22.

The reaction vessel shown in the drawings can be prepared for use in the method of this invention by immobilizing one or more reactants on one or more of the surfaces 2 and 22.

Advantages of the invention are further illustrated in the following examples.

General

Throughout this note, reference will be made to solid phases which are coated with antibody. The coating substrate in all examples is polystyrene, and the antibody is attached to the polystyrene by incubating the polystyrene in a solution of antibody overnight at room temperature. The coated polystyrene is rinsed in buffer before use. Buffer composition is as follows:-

Per 100ml of buffer

Tween 20 (Registered Trade Mark) 0.05g
sodium chloride 0.8g
potassium chloride 0.02g
disodium hydrogen phosphate (anhydrous) 0.115g
potassium dihydrogen phosphate 0.02g

Examples are classified as series A and series B.

Series A examples are listed as evidence in support of the proposition that the rate of reaction of solution phase antigen with solid antibody increases as the capillary dimension decreases. The capillary dimension is defined as that distance $X$ such that all the solution phase is less than distance $X$ from the nearest point on the coated solid phase. It should be noted that the capillary dimension for a system in which both surfaces are coated is half the capillary dimension of a similar system in which only one of the surfaces is coated.

Series B examples are listed as evidence in favour of the proposition that the claimed rate enhancement occurs over a wide range of antibody-antigen systems.

## Series A Examples

### Example A1:

The coated solid phase is a polystyrene microtitre well which measures 6mm across.

When fluid is placed into the microtitre well, the capillary dimension is 3mm.

The coating antibody is sheep anti-human IgG.

The solution phase antigen is $^{125}$I-labelled human IgG.

### Procedure

Solution phase antigen was placed in a series of coated wells and decanted at various times. Wells were washed and the amount of antigen bound to the wells was measured in a gamma counter. Results are plotted on the attached graph of percent maximum binding as a function of time (curve A1 in Fig. 5.) The time to maximum binding is at least 180 minutes.

### Example A2:

The coated solid phase is a polystyrene rod with an outer diameter of 2.8mm. Fluid is placed into a flat-bottomed glass cylinder of inner diameter 4.0mm. When the rod is inserted in the cylinder, the capillary dimension is 0.6mm.

The coating antibody is sheep antihuman IgG.

The solution phase antigen is$^{125}$I-labelled human IgG.

### Procedure

As for example A1 except that the capillary space was formed as described above.

Results are plotted as for Example A1 (curve A2 in Fig. 5). The time to maximum binding is 90 minutes (at least).

### Example A3:

As for example A2 except that the coated polystyrene rod is replaced with a coated polystyrene plunger and the flat-bottomed glass cylinder is replaced with an uncoated polystyrene bottle, or complementary shape to the plunger as in Figs, 1 and 2 of the drawings.

The capillary space is 0.05mm.

Results are plotted as above (curve A3 in Fig. 5)

The time to maximum binding is about 15 minutes.

## Series B Examples

These examples were all performed using the plunger/bottle system of Figs. 1 and 2.

The purpose of this series is to show that the rapid incubation times (15 minutes or less) are achievable in a range of antibody-antigen systems and in a range of assay formats.

### Examples B1 = Example A3

Antigen : $^{125}$I-labelled human IgG(solution phase)
Antibody : sheep anti human IgG, (immobilised)
Assay Format : RIA (radioimmunossay)
For assay details see example A3.
The incubation time is fifteen minutes.

### Example B2

Antigen : Rotavirus (solution phase)
Antibody : mouse monoclonal anti rotavirus (immobilised)
Assay Format : Enzyme immunoassay.
The assay is conducted as follows:-

Plungers were coated with anti-rotavirus antibody. A solution phase comprising rotavirus and anti-rotavirus-antibody enzyme conjugate was added to the wells. The enzyme was horse radish peroxidase (HRPO). The plungers were then added to the bottles and removed after various period of time and rinsed. The rinsed plungers were measured for the amount of bound enzyme as follows:

The plungers were inserted into new bottles which contained substrate solution for the HRPO. After five minutes plungers were removed and the degree of color development measured on a spectrophotometer.

Maximum colour development for a given quantity of rotavirus occurred after a first incubation period of about 10 minutes. The degree of colour development after 10 minutes was proportional to the amount of rotavirus present.

EXAMPLE C

The purpose of this example is to show that the plunger/bottle system of Figs. 1 and 2 can be assembled in a way that allows several samples to be analysed simultaneously.

Plungers were inserted into holes drilled in a strip of rigid plastic sheet. Up to 10 plungers were placed in line.

Bottles were held in a similar strip of plastic sheet in 2 rows of up to 10 bottles each row, the spacing of the bottles coinciding with the spacing of the plungers.

Other conditions were as described in example B2.

Up to 10 samples could be analysed at the same time.

This example illustrates how many samples may be analysed at the same time and it will be appreciated that an integrally formed construction having a plurality of plunger and another integrally formed construction having a similar plurality of bottles might be made to facilitate analysis on a large scale.

The entire contents of the provisional specifications lodged with Australian Patent Applications of which this is the complete specification are hereby imported into this specification and form part of the disclosure of this specification. The claims form part of the disclosure of this specification.

**Claims**

1. A reaction vessel comprising: a first body having a cavity therein extending to the exterior thereof and a second body having a portion adapted to be received in said cavity, wherein said cavity conically taperingly narrows towards the inner end of the cavity with an included conic angle of less than 140°, wherein said portion conically taperingly narrows towards an end of said second body with an included conic angle of less than 140°, wherein the cavity and said portion are so similarly shaped as to define, and wherein one of the cavity and said portion has projections thereon adapted to space apart said first body and said second body when said second body is received within said cavity so as to define, when said portion is in a juxtaposition within said cavity, a capillary space between said first body and said second body having a thickness of not greater than 1.00mm.

2. A reaction vessel as claimed in claim 1, wherein said capillary space has a volume, when in use, of not more than 50 microlitres.

3. A reaction vessel comprising a first body having a cavity therein extending to the exterior thereof and a second body having a portion adapted to be received in said cavity, wherein the cavity has a first part adjacent the exterior of the first body which conically taperingly narrows towards the inner end of the cavity with an included conic angle of less than 140°, and a second part extending to the inner end of the cavity which conically taperingly narrows towards the inner end of the cavity with an included conic angle of from 30° to 175°, wherein said portion has a first part remote from an end of said portion which conically taperingly narrows towards said end of said portion with an included conic angle of less than 140°, and a second part extending to said end of said portion which conically taperingly narrows towards said end of said portion with an included conic angle of from 30° to 175°, wherein the first part of said cavity and said first part of said portion are so similarly shaped such as to define, and wherein one of said first part of said cavity and said first part of said portion has projections thereon adapted to space apart said first part of said cavity and said first part of said portion when said portion is received within said cavity so as to define, when said portion is in a juxtaposition within said cavity, a capillary space between said first part of the cavity and said first part of said portion having a thickness of not greater than 1.0mm.

4. A reaction vessel as claimed in claim 3, wherein the conic angle of said first part of said cavity and said first part of said portion is less than the conic angle of said second part of said cavity and said second part of said portion.

5. A reaction vessel as claimed in claim 3 or 4, wherein the inner end of said cavity and said end of said portion are generally frusto-conical or rounded and thereat have a diameter of from 0.5 to 2.5mm.

6. A reaction vessel as claimed in claim 3,4 or 5, wherein said projections are located on said first part of said portion.

7. A reaction vessel as claimed in claim 3,4,5 or 6, including an overflow space provided in the first body to receive liquid displaced from said cavity when said second body is introduced into said cavity.

8. A method of carrying out chemical or biological reactions utilizing a reaction vessel comprising: a first body having a cavity therein extending to the exterior thereof and a second body having a portion adapted to be received in said cavity, wherein said cavity conically taperingly narrows towards the inner end of the cavity with an included conic angle of less than 140°, wherein said portion conically taperingly narrows towards an end of said second body with an included conic angle of less than 140°, wherein the cavity and said portion are so similarly shaped as to define, and wherein one of the cavity and said portion has projections thereon adapted to space apart said first body and said second body when said second body is received within said cavity so as to define, when said portion is in a juxtaposition within said cavity, a capillary space between said first body and said second body having a thickness of not greater than 1.0mm, which comprises locating said second body in said cavity in said juxtaposition to define said capillary space wherein a reactant has been immobilized on one of the surface of said cavity and said second body, defining said capillary space prior to them being so juxtaposed and interposing into said capillary space a co-reactant.

9. A kit for chemical or biological reactions, the kit comprising a reaction vessel comprising: a first body having a cavity therein extending to the exterior thereof and a second body having a portion adapted to be received in said cavity, wherein said cavity conically taperingly narrows towards the inner end of the cavity with an included conic angle of less than 140°, wherein said portion conically taperingly narrows towards an end of said second body with an included conic angle of less than 140°, wherein the cavity and said portion arew so similarly shaped as to define, and wherein one of the cavity and said portion has projections thereon adapted to space apart said first body and said second body when said second body is received within said cavity so as to define, when said portion is in a juxtaposition within said cavity, a capillary space between said first body and said second body having a thickness of not greater than 1.0mm, and wherein a reactant is immobilized on one of the surfaces of said cavity and said second body which in use define said capillary space.

10. A kit as claimed in claim 9, including different reactants immobilized in different areas of said surfaces.

0 245 994

_ⅡG_. _1_ .

_ⅡG_. _2_ .

_ⅡG_ . _3_ .

_ⅡG_ . _4_ .

FIG. 5.